# EUROPEAN PATENT SPECIFICATION

(11) **EP 3 974 825 B1**
(45) Date of publication and mention of the grant of the patent: **18.06.2025**
(21) Application number: 19929483.6
(22) Date of filing: 15.07.2019
(51) Int. Cl.: G01N 30/02, G01N 30/06, G01N 1/38, G01N 1/40, G01N 1/30

(54) **METHOD FOR EXTRACTING LIPIDS IN FOOD**
VERFAHREN ZUR EXTRAKTION VON LIPIDEN IN NAHRUNGSMITTELN
PROCÉDÉ D'EXTRACTION DE LIPIDES DANS DES ALIMENTS

(30) Priority: 20.05.2019 CN 201910420276
(43) Date of publication of application: 30.03.2022
(73) Proprietor: Beijing Sanyuan Foods Co., Ltd., Beijing 100163 (CN)
(72) Inventor: CHEN, Lijun, Beijing 100163 (CN); ZHAO, Junying, Beijing 100163 (CN); JIANG, Tiemin, Beijing 100163 (CN); QIAO, Weicang, Beijing 100163 (CN); LIU, Qian, Beijing 100163 (CN); LIU, Yan, Beijing 100163 (CN); ZHANG, Minghui, Beijing 100163 (CN); WANG, Xiaodi, Beijing 100163 (CN); ZHANG, Ying, Beijing 100163 (CN)
(74) Representative: LLR
(86) International application number: PCT/CN2019/095969
(87) International publication number: WO 2020/232813

(56) References cited:
- WO-A1-2010/062197
- CN-A- 107 632 095
- CN-A- 109 001 360
- CN-A- 109 081 850
- CN-A- 109 444 316
- FONG B ET AL: "Liquid chromatography-high-resolution electrostatic ion-trap mass spectrometric analysis of GD"3 ganglioside in dairy products", INTERNATIONAL DAIRY JOURNAL, ELSEVIER APPLIED SCIENCE, BARKING, GB, vol. 21, no. 1, 1 January 2011 (2011-01-01), pages 42 - 47, XP027450123, ISSN: 0958-6946, DOI: 10.1016/J.IDAIRYJ.2010.07.001
- JENSEN R G: "THE LIPIDS IN HUMAN MILK", PROGRESS IN LIPID RESEARCH, PERGAMON PRESS, PARIS, FR, vol. 35, no. 1, 1 January 1996 (1996-01-01), pages 53 - 92, XP007904238, ISSN: 0163-7827, DOI: 10.1016/0163-7827(95)00010-0
- SHE YUQI ET AL: "An analysis on the suppression of NO and PGE2by diphenylheptane A and its effect on glycerophospholipids of lipopolysaccharide-induced RAW264.7 cells with UPLC/ESI-QTOF-MS", ANALYTICAL AND BIOANALYTICAL CHEMISTRY, SPRINGER BERLIN HEIDELBERG, BERLIN/HEIDELBERG, vol. 408, no. 12, 30 March 2016 (2016-03-30), pages 3185 - 3201, XP035867700, ISSN: 1618-2642, DOI: 10.1007/S00216-016-9383-5
- 周佩佩 (ZHOU, PEIPEI): "海藻中脂肪酸的提取和分析方法研究 (Study on Extraction and Analysis of Fatty Acids in Seaweeds)", 中国优秀硕士学位论文全文数据库 (CHINESE MASTER’S THESES FULL-TEXT DATABASE), 31 May 2017 (2017-05-31), XP55756620, DOI: 20200114085452Y

## Description

### Technical Field

The present invention relates to the field of food extraction and detection, in particular to a method for extracting lipids in food and a method for detecting lipids in food.

### Background Art

Lipids are the primary nutrients in food, which mainly include eight classes: fatty acids, glycerolipids (including triglycerides, diglycerides and monoglycerides), glycerophospholipids (phosphatidylcholine (PC), phosphatidylethanolamine (PE), phosphatidylinositol (PI), phosphatidylserine (PS), phosphatidylglycerol (PG), phosphatidic acid (PA)), sphingolipids (ceramide (Cer), sphingomyelin (SM)), saccharolipids (gangliosides (GM, GD)), sterol lipids, prenol lipids, and polyketides. Since the concept of lipidomics was proposed in 2003, the importance of food lipidomics to health and disease prevention has been gradually recognized.

Breast milk lipids are the second largest component in human breast milk after lactose, which mainly include glycerolipids, glycerophospholipids, sterol lipids and saccharolipids, and their content in colostrum is up to 40 g/L. Breast milk not only provide 40-55% energy for the growth and development of infants who are exclusively breastfed, but also provides phospholipids which are indispensable components of cell and organelle membrane structures and have the function of resisting various metabolic diseases, and gangliosides which are important components for forming cerebral lipids and cerebral cortex. The breastfeeding rate in China is less than 29%, and the strong support of the data about the composition of breast milk can be one of the effective ways to promote breastfeeding, and the composition of breast milk is also the gold standard for designing infant formula. Since mother's genes, diet, environment, and other factors can influence breast milk lipids, it is necessary to detect representative breast milk samples from different regions, different lactation periods, different races, etc. for determining the categories and contents of breast milk lipids, so as to establish a breast milk lipid database. However, breast milk is the only food for infants, especially for those under six months of age who are exclusively breastfed. Therefore, it is difficult to collect breast milk samples, and the collected sample volume is small. Therefore, the establishment of the breast milk lipid database depends on the high-throughput detection method, which not only increases the detection throughput with small breast milk sample, but also ensures the safety of the testers in the process of sample pretreatment and detection. Therefore, the establishment of a high-throughput, low sample and safe detection method for breast milk lipids is a prerequisite for analyzing breast milk lipids and thus promoting breastfeeding and developing more nutritious and healthy infant formula.

When designing various solid and liquid foods, lipid composition is an important aspect of their nutritional function. Especially for designing more scientific and reasonable infant formula foods, it is necessary to detect the lipid composition in the existing infant formula foods, and perform qualitative and quantitative analysis on the lipid composition in different infant food ingredients. High-throughput and safe detection technologies are also needed.

At present, the methods for detecting lipid in foods, especially breast milk, dairy products and ingredients are mainly based on detection of different targets of glycerolipids, phospholipids (including glycerophospholipids and sphingomyelins), fatty acids and gangliosides using different instruments after pretreating samples by different methods respectively. Wherein, the methods for pretreating samples mainly include conventional Folch method and Bligh and Dyer method for extracting glycerolipids or phospholipids:

### 1) Folch method

The sample is diluted 25 times. 2 g milk, 50 g chloroform/methanol (2:1) and 5 mL NaCl (0.05 M) are mixed by shaking at 200 rpm for 20 min, centrifuged at 5,000 rpm for 20 min. The thus obtained bottom chloroform layer is removed, and 35 mL of chloroform is added to the upper methanol layer. The above process is repeated and the obtained bottom chloroform layers are combined and then evaporated to dryness, and stored at -20°C for later use. The neutral glycerolipids is separated from polar phospholipids by solid phase extraction column.

The specific process for solid phase extraction includes: dissolving 50 mg lipid extract in 1 mL of mixture of chloroform/methanol (95:5, v/v), then adding the thus obtained solution to silica gel column (500 mg) which has been activated with 5 mL of chloroform/methanol (95:5, v/v) in advance, wherein triglycerides is eluted with 10 mL of chloroform/methanol (95:5, v/v), and phospholipids is eluted with mixed solution of 5 mL of methanol and 5 mL of chloroform/methanol/water (3:5:2, v/v/v), evaporating the eluents to dryness, and redissolving the thus obtained products into chloroform/methanol (2:1, v/v) for detection.

The following elution method can also be employed: dissolution of lipid: dissolving 200 µL of the extracted lipid in 1 mL of chloroform: methanol (v/v=2:1); activation of silica gel column: activating the silica gel column (6 mL, 1 g adsorbent) with 3 mL of n-hexane, discarding the eluent, and then pouring the redissolved lipid solution into the column; elution of nonpolar lipids: firstly, adding 3 mL of n-hexane/diethyl ether (8:2, V/V) solution to the column and collecting the eluent, followed by adding 3 mL of n-hexane/diethyl ether (1:1, V/V) solution and collecting the eluent; after blowing dry with nitrogen, redissolving the thus obtained residue for detection; elution of phospholipids: adding 4 mL of methanol to the column, followed by adding 2 mL of methanol and 2 mL of dichloromethane/methanol/water (3:5:2, V/V/V), collecting the eluent, after blowing dry with nitrogen, redissolving the thus obtained residue for detection.

### 2) Bligh and Dyer method

This method has not been reported for lipid extraction from breast milk, and is mainly used to extract lipids from animal dairy products. Specifically, 18.7 mL of chloroform-methanol (V/V=1:2) is added to 500 µL of milk, followed by adding 0.62 mL of chloroform and 0.62 mL of water, the thus obtained mixture is centrifuged after vortex shaking, the bottom organic phase is taken for concentration under nitrogen to obtain lipids.

### 3) The method for extracting ganglioside is mainly performed as follows:

8 mL of methanol, 4 mL of chloroform and 1 mL of water are added to 2 mL of milk, the thus obtained mixture is mixed under sonication for 5 min and centrifugated at 8800 g for 5 min; 2 mL of water is added thereto and the upper layer is collected after layering, then adding 6 mL of methanol, 3 mL of chloroform and 2 mL of water to the residue, the upper layer is collected after sonication and centrifugation, and combined with the previously collected one. The combined upper layer is freeze-dried under rotation, redissolved with 1 mL of 1:1 methanol: water, and enriched with a solid phase extraction column.

The instruments and methods for detecting lipids in food, especially breast milk and infant formula powder and ingredients are as follows. Gas chromatography-mass spectrometry (GC-MS) is used to detect fatty acids; liquid chromatography(LC) or supercritical fluid chromatography (SFC) combined with quadrupole-time of flight-mass spectrometer (Q-TOF-MS) is used to detect triglycerides and the composition of fatty acids thereof; liquid chromatography with evaporative light detector, thin layer chromatography or LC- Q-TOF-MS is used to detect phospholipids and the composition of fatty acids thereof; tandem mass spectrometry or LC-Q-TOF-MS is used to detect gangliosides. Various detection methods are specifically as follows.
1) when analyzing glycerolipids in breast milk by SFC- Q-TOF-MS, the main parameters of mass spectrometry are as follows: in positive ion mode; scanning range: 50-2000 m/z; flow rate of nitrogen for desolvatinon: 800 L/h; collision gas: argon; ion source temperature: 120°C; desolvation temperature: 450°C; capillary voltage: 3.0 kV; sampling cone voltage: 25 V; flow rate of sampling cone gas: 20 L/h; low collision energy: 4 eV; high-gradient collision energy: 20-60 eV.
2) At present, a liquid chromatography fluorescence detector is widely used to detect phospholipids, but this method cannot determine the composition of fatty acids in phospholipids. Alternatively, MS/MS ^{ALL} mode of Triple-TOF-MS is used to analyze phospholipids, wherein, the detection conditions are as follows: in positive and negative ion modes, collision energy: 45eV; voltage: 0.96 kV; back pressure: 1.25 psi. And it has been reported that LC-MS-IT-TOF is used to analysize phospholipids in breast milk, wherein, the liquid phase parameters are as follows: Hilic chromatographic column: 150×4.6 mm I.D., 2.7 µm d.p.; mobile phase A: acetonitrile-ammonium formate (9:1, v/v; pH=5.5); phase B: acetonitrile-methanol-ammonium formate (55:35:10, v/v/v; pH=5.5); elution gradient: 0-10 min, 40% B; 15 min, 45%B; 25 min, 80% B; 40 min, 90% B; flow rate: 0.7 mL/min, injection volume: 5 µL; the parameters of mass spectrometry are as follows: in positive ion mode and negative ion mode, curved desolvation tube temperature: 200 °C, ion source temperature: 200 °C, N2 spraying gas flow rate: 1.5 L/min, scanning range: 200-1200 m/z, ion accumulation time: 100 ms; secondary scanning: scanning range: 200-1000 m/z, ion accumulation time: 30 ms; parent ion separation parameters: width: 3, time: 20 ms, 600-1000 m/z; collision dissociation parameters: energy: 100%, collision gas: 100%, time: 50 ms.
3) The parameters of LC-Q-TOF-MS for analysis of gangliosides are as follows: liquid phase conditions: chromatographic column: BEH C18 column (1.7×150 mm, 2.1mm i.d), column temperature: 50°C; mobile phase A: water/methanol/ammonium acetate (1 mmol/L) (90/10/0.1 v/v/v), mobile phase B: methanol/ammonium acetate (1 mmol/L) (100/0.1 v/v); elution gradient: 0 min, 10% A; 0.2 min, 10% A; 8.2 min, 5% A; 12.2 min, 5% A; 12.4 min, 0% A; 18.4 min, 0%A; 18.6 min, 10%A; 21min, 10%A,; flow rate: 0.2 mL/min. The mass spectrometry conditions are: ion spray voltage: 4000 V; ion source temperature: 400 °C, de-clustering voltage: 40 V, ion source gas 1: 40 psi, ion source gas 2: 35 psi, curtain gas: 15 psi, collision energy: 40 V.

Shotgun method was proposed in 2004, in which the extracted lipids are directly and simultaneously taken for ionization by mass spectrometry and detection. This mehtod offers fast analysis, however, the sample needs to be diluted about 200 times in order to prevent the saturation of the detector and pollution of the mass spectrometry, and it is hard to analyze low-concentration lipids qualitatively or quantitatively due to the ion inhibition of high-abundance or easily ionized lipids.

### Disadvantages in prior art

Although LC- Q-TOF-MS can be speculated to detect triglycerides and the composition of fatty acids thereof, phospholipids and the composition of fatty acids thereof, and gangliosides in foods, especially breast milk, maternal and infant milk and ingredients at the same time, there are still disadvantages in mass sample detection for establishment of the lipid databases of breast milk and different raw materials in the existing methods:
1) Different lipids need to be extracted separately.
   Glycerolipids and phospholipids can be detected by treating the sample only once, i.e., they can be detected after being separated nonpolar glycerolipids from polar glyceryl phosphatides through solid-phase extraction column. However, for gangliosides, it is necessary to take an additional sample for pretreatment.
2) Chloroform, which is a highly toxic substance, needs to be used for lipid extraction.
   Whether Folch method or Bligh and Dyer method for extracting glycerolipids and phospholipids, or the existing methods for extracting gangliosides, chloroform which is a highly toxic substance is used. When exposed to light, chloroform will react with oxygen in air, and gradually decompose to produce highly toxic phosgene which has an anesthetic effect and is harmful to heart, liver, and kidney, and is very harmful to human health in the process of mass sample detection.
3) Different instruments need to be used for detecting glycerolipids, phospholipids and gangliosides. At present, although phospholipids and glycerolipids can be detected simultaneously by shotgun method, on one hand, the sample needs to be diluted 200 times, which makes it difficult to detect some low-concentration lipids; and on the other hand, gangliosides need to be analyzed by LC-Q-TOF-MS. As reported in the existing literatures, in widely used methods, phospholipids are detected by high performance liquid chromatography fluorescence detector, glycerolipids and gangliosides are detected by liquid chromatography-mass spectrometry, and the composition of fatty acid composition in samples is detected by gas chromatography-mass spectrometry. Therefore, at least two sets of instruments, mass spectrometry and LC-MS, are used in series to analyze lipids in food. FONG B ET AL: "Liquid chromatography-high-resolution electrostatic ion-trap mass spectrometric analysis of GD3 ganglioside in dairy products", INTERNATIONAL DAIRY JOURNAL, ELSEVIER APPLIED SCIENCE, BARKING, GB, vol. 21, no. 1, 1 January 2011, pages 42-47, XP027450123, describes a lipid extraction solvent which comprises chloroform.

HPLC-MS is the main technology for detecting milk-derived lipids at molecular level presently, and the summary of researches on food lipid detection based on this technology is shown in Table 1, there is still an urgent need for a complete set of high-throughput detection technologies for qualitative and quantitative analysis of phospholipids and glycerolipids at the same time. There is only one report on qualitative analysis of phospholipids and glycerolipids at the same time, however, it is silent about the detection of gangliosides and quantitative analysis.

**Table 1: Summary of detection of molecular compositions of milk-dervied glycerolipids and phospholipids based on LC-MS technology in literatures**

| Sample number | Pretreatment method | Detection technology | Detection categories | Qualitative and quantitativ e |
|---|---|---|---|---|
| 73 | Folch method with minor modification | UPLC-Triple-TOF-MS/MS | Phospholipid s: PC, LPC, PS, PE, SM, PI | Qualitative and quantitativ e |
| 18 | Folch method with minor modification | LC-ESI-IT-TOF-MS | Phospholipid s: PG, LPG, LPE, PI, LPI, PC, LPC, PS, PA, SM | Qualitative and quantitativ e |
| 20 | Folch and Bligh methods | LC-Q-Exactive Orbitrap Mass Spectrometry | Phospholipid s: SM, LPC, PC, PE, PG, PI, PA, PS, LPE; other lipids: DG, Cer | Qualitative and quantitativ e, and each type is quantified by external standard method |
| 5 | Folch method + solid phase extraction | LC-ESI-Q-TOF-MS | Phospholipid s: PC, PE, PI, PS, SM, LPC | Qualitative , relative quantificati on % |
| 45 | Folch method | LC-APCI-MS | Glycerolipids : TAG, DAG | Qualitative , LC-ELSD quantitativ e |
| 168 | Chloroform/methanol/w ater (4/8/3) | LC-ESI-TSQ quadrupole mass spectrometry | Phospholipid s: PI, PE, PC, PS, SM, LPC, LPE | Qualitative , and quantificati on by external standard method |
| 40 | Modifed Folch method | LC-MS | Glycerolipids : TAG, DAG | Qualitative |
| 32 | Folch method | LC-LTQ Orbitrap Velos Mass Spectrometry | Phospholipid s and other lipids: PC, PE, PS, SM, PI, LPC, Cer | Qualitative , and quantificati on by external standard method |
| 12 | Folch method | LC-4500 QTRAP | Phospholipid s: PE, PS, PI, PC, SM Other lipids: ST, Cer | Qualitative , C-ELSD quantitativ e |
| 8 | Folch method | LC-ESI-MS | Phospholipid s: PI, PE, PC, PS, SM, LPC | Qualitative , LC-ELSD quantitativ e |
| 1 | Solid phase extraction | LC-6540 Q-TOF-MS (ESI) | Glycerolipids and fatty acids: FA, TG, DG | Qualitative |
| | | | Phospholipid s and other lipids: PE, PC, SM, ST | |
| 2 | Folch method + solid phase extraction | LC-MALDI-TOF/TOF-MS | Phospholipid s: PC, SM, PE, PS, LPC ,PI | Qualitative |
| | Ethanol/diethyl ether/petroleum ether (2/5/5) | LC-LTQ-Orbitrap XL hybrid Mass Spectrometry | Glycerolipids : TAG | Qualitative and quantitativ e |

To sum up, in the prior art, phospholipids and glycerolipids are mostly qualitatively and quantitatively determined at the molecular level, respectively, which need to seperate lipid from different samples for detection separately, resulting in mass sample consumption, multi-time pretreatment operations, time-consuming, labor-intensive and material-consuming. Phospholipids are mostly quantified at the sub-class level by the evaporative light detector, that is, only phosphatidylcholines can be quantified, and it is impossible to determine the contents of phosphatidylcholines with different fatty acid chains.

### Summary of the Invention

### Object of the invention

Based on the characteristics of food lipids and the principle of detecting food lipids by LC-Q-TOF-MS, the present invention aims to establish a safe, high-throughput method with low sample, wherein, low sample and safer lipid extraction reagents are used, extract of different lipids can be obtained by one-time treatment, which then can be detected qualitatively and quantitatively, simultaneously or separately, thereby reducing sample usage, improving safety, reducing cost and increasing efficiency. The method can be applied to qualitatively and quantitatively analyze the lipid composition, establish the lipid database of food, especially breast milk, and provide technical support for the scientific design of food lipids, especially maternal and infant food lipids.

### Technical solutions of the invention

The present invention provides a method according to claim 1 for extracting lipids in food.

In some embodiments, the present invention provides a method for extracting lipids in food, which can extract lipids in food, including fatty acids, glycerolipids, phospholipids comprising glycerophospholipids and sphingomyelins, and gangliosides.

The method comprises steps of: mixing a food sample with mixed internal standard; mixing the mixture of the food samples and the mixed internal standard with lipid extraction solvent and ultrapure water; centrifuging the mixture obtained in above mixing step; repeating the mixing step and the centrifuging step for several times; combining the upper phase comprising gangliosides which are collected after each centrifugations, and combining the lower organic phase comprising phospholipids, fatty acids, and glycerolipids which are collected after each centrifugations.

The lipid extraction solvent is a mixture of dichloromethane and methanol.

Wherein, the food sample can be a solid or liquid food that can be eaten directly, or a solid food that can be eaten after being brewed into liquid form. In case of a solid or liquid food that can be eaten directly, the solid or liquid food can be directly used for extracting lipid. In case of a solid food that can be eaten after being brewed into liquid form, the solid food can be used for extracting lipid after being brewed into liquid form according to the brewing method for packaged food.

In the method for extracting lipids in food of the present invention, the sample size of food used can only be 50-350 µL of liquid food samples, or liquid prepared according to the brewing method for packaged food, or 0.05-0.1 g of powdered solid food samples. Lipids can be extracted from a small sample size of food, which provides technical support for subsequent lipid detection and content determination, and has the advantages of wide applicability and less sample consumption.

Wherein, the mixed internal standard is one or more of deuterated glycerolipids, different types of deuterated phospholipids and different types of deuterated sphingolipids. The different types of deuterated phospholipids and different types of deuterated sphingolipids mean that their types are different from those of phospholipids and sphingolipids in food samples. For example, the mixed internal standard may include one or more of the followings: glycerolipids, phosphatidylcholine, phosphatidylserine, phosphatidylinositol, phosphatidylethanolamine, phosphatidylglycerol, phosphatidic acid, sphingomyelin, ceramide, etc. The mixed internal standard can eliminate the disadvantage that the data from different testers and different batches of samples are difficult to analyze collectively due to operational differences and instrument state differences, and has the advantages of improving data comparability and accuracy.

The principle for determining the amount of mixed internal standard is as follows: the mass concentration of each lipid after adding the mixed internal standard is 60-140%, preferably 80-120% of the same type of lipid in the original sample, more preferably, the mass concentration of each lipid after adding the mixed internal standard is equivalent to that of the same type of lipid in the original sample; namely, the results of food sample detection are employed to determine the content of each lipid through the external standard curve, wherein the amount of each lipid added in the mixed internal standard is 60-140%, preferably 80%-120%, more preferably 100% of the content of each lipid determined through the external standard curve.

According to the present invention, dichloromethane is used instead of chloroform in the process of lipid extraction, separation, and purification to improve the safety of the method, which is the least toxic among methane chlorides, and has a toxicity only 0.11% of that of tetrachloromethane. At the same time, it is intersoluble with lipids and thus can be applied for lipid extraction, separation, and purification.

According to the present invention, the method for extracting lipids in food comprises the following steps: performing the first centrifugation by using the mixture of dichloromethane and methanol as lipid extraction solvent to obtain an upper phase and a lower organic phase. The volume ratio of dichloromethane, methanol and ultrapure water is 1-3.5:1-3:0.2-0.55, preferably 1-3:1-3:0.2-0.55, more preferably 9:10:2. After centrifugal separation with dichloromethane, methanol and ultrapure water in the above ratios, gangliosides can be separated into the upper phase, so as to sufficiently extract lipid and separate fatty acids/glycerides/phospholipids from gangliosides. According to the present invention, dichloromethane is used in an amount of 0.5-2 mL/100 µL liquid food sample or 1-3 mL/0.05 g powdered solid food sample, preferably 0.6-1.2 mL/100 µL liquid food sample or 1.5-2.5 mL/0.05 g powdered solid food sample. When dichloromethane is used in such amount, lipids can be sufficiently extracted and different lipids can be separated.

In one embodiment of the present invention, the ultrapure water and dichloromethane are added in two portions, and the methanol is added all at once. In one embodiment of the present invention, the food sample and the mixed internal standard are mixed, ultrapure water, dichloromethane and methanol are added thereto, followed by shaking, and then the remaining ultrapure water and dichloromethane are added. In one embodiment of the present invention, the amount of ultrapure water and dichloromethane added in two portions is the same. In one embodiment of the present invention, the amount of ultrapure water and dichloromethane added in two portions is different. In one embodiment of the present invention, the ratio of ultrapure water added in two portions is 0.15-0.4: 0.1-0.3; more preferably 0.2:0.25. In one embodiment of the present invention, the ratio of dichloromethane added in two portions is 0.8-1.0: 0.85-1.1; more preferably 0.9:1.0. In one embodiment of the present invention, ultrapure water, dichloromethane, and methanol are added, followed by shaking and carrying out sonication, and then the remaining ultrapure water and dichloromethane are added thereto. In one embodiment of the present invention, the sonication is carried out under the following conditions: 20-50 KHz, 600-900 W, 20-30 °C, 1-10 minutes; preferably 40 KHz, 800 W, 25 °C, 5 minutes.

According to the present invention, the method for extracting lipids in food further comprises the following steps: adding dichloromethane to the upper phase obtained by the first centrifugation for performing the second centrifugation to obtain an upper phase and a lower organic phase. Dichloromethane is used in an amount of 0.2-1.8 mL/100 µL liquid food sample or 1-3 mL/0.05 g powdered solid food sample. In one embodiment of the present invention, dichloromethane is used in an amount of 0.6-1.2 mL/100 µL liquid food sample or 1.5-2.5 mL/0.05 g powdered solid food sample. Through the second separation above, fatty acids/glycerolipids/phospholipids in the sample can be sufficiently extracted into the organic phase, thus separating different lipids.

According to the present invention, the method for extracting lipids in food further comprises the following steps: adding ultrapure water, dichloromethane and methanol to the lower organic phase obtained by the first centrifugation for performing the third centrifugation to obtain an upper phase and a lower organic phase. The volume ratio of dichloromethane, methanol and ultrapure water is 0.3-1:1-3.5:0.5-3, preferably 0.3-0.6:1-2:0.5-1. Dichloromethane is used in an amount of 0.3-1 mL/100 µL liquid food sample or 0.3-1 mL/0.05 g powdered solid food sample. Through the third separation above, gangliosides remained in the organic phase can be sufficiently extracted into the aqueous phase, thus sufficiently extracting gangliosides.

According to the present invention, the method for extracting lipids in food further comprises the following steps: combining the upper phase obtained by the second centrifugation with the upper phase obtained by the third centrifugation, and drying the combined upper phases with nitrogen to obtain gangliosides. According to the present invention, by limiting specific lipid extraction solvent, the gangliosides are separated into the upper phase and separated from fatty acids/glycerolipids/phospholipids to obtain an extraction solution containing only gangliosides, thus achieving efficient separation. The drying can be by blow drying.

In one embodiment of the present invention, the method for extracting lipids in food further comprises the step of purifying the gangliosides. In one embodiment of the present invention, the steps of purifying the gangliosides are specifically as follows: dissolving the obtained gangliosides in 60-90% methanol solution, followed by centrifugation, then taking the upper phase for drying with nitrogen to obtain the gangliosides. In one embodiment of the present invention, methanol solution is used in an amount of 100-350 µL/100 µL liquid food sample or 100-350 µL/0.05 g powdered solid food sample. Through the above purification steps, the gangliosides obtained by the present invention do not contain precipitated impurities such as proteins generated in the extraction process, thus achieving the effect of preventing impurities from entering the liquid system.

According to the present invention, the method for extracting lipids in food further comprises the following steps: combining the lower organic phase obtained by the second centrifugation with the lower organic phase obtained by the third centrifugation, and drying the combined organic phases with nitrogen to obtain phospholipids, fatty acids, and glycerolipids The phospholipids, fatty acids and glycerolipids obtained according to the present invention do not contain insoluble impurities, such as protein precipitations, thus achieving the effect of protecting the liquid system. The drying can be by blow drying.

In one embodiment of the present invention, the method for extracting lipids in food further comprises the steps of purifying the phospholipids, fatty acids and glycerolipids. In one embodiment of the present invention, the step of purifying the phospholipids, fatty acids and glycerolipids are specifically as follows: dissolving the obtained phospholipids, fatty acids and glycerolipids in a solution of ammonium acetate in dichloromethane and methanol, followed by centrifugation, then taking the lower organic phase for blowing dry with nitrogen to obtain the phospholipids, fatty acids, and glycerolipids. In one embodiment of the present invention, the ratio of dichloromethane and methanol in the solution of ammonium acetate in dichloromethane and methanol is 0.6-1.5:0.6-1.2; more preferably 1:1. In one embodiment of the present invention, the concentration of ammonium acetate is 5-15 mmol/L, preferably 8-12 mmol/L, more preferably 10 mmol/L. In one embodiment of the present invention, the solution of ammonium acetate in dichloromethane and methanol is used in an amount of 0.2-2.5 mL/100 µL liquid food sample or 0.5-2.5 mL/0.05 g powdered solid food sample.

According to conventional Folch method, after dilution, lipids are extracted from a sample by using chloroform: methanol =2:1 as solvent, the lower organic phase is removed after centrifugation, chloroform is added to the upper phase for the secondary extraction. In the present invention, dichloromethane and methanol with a specific volume ratio are used as the lipid extraction solvent, in order to separate gangliosides in the liquid sample into the upper phase; the obtained lower organic phase and upper phase are separated again as follows: extracting gangliosides from the organic phase again with dichloromethane, methanol and water in a specific volume ratio, and extracting fatty acids, glycerolipids and phospholipids from the aqueous phase again with dichloromethane.

According to the present invention, the method for extracting lipids in food further comprises the following steps: dissolving the obtained phospholipids, fatty acids and glycerolipids in the solution of dichloromethane and methanol with a volume ratio of 0.6-1.5:0.6-1.2 (preferably 2:1) to obtain a redissolved lipid solution, and then separating the redissolved lipid solution with a silica gel column to obtain glycerolipids/fatty acids and phospholipids, respectively. IThe solution of dichloromethane and methanol is used in an amount of 0.2-2.5 mL/100 µL liquid food sample or 0.5-2.5 mL/0.05 g powdered solid food sample.

In another embodiment of the present invention, separation with the silica gel column specifically comprises the following steps:
activation of silica gel column: the silica gel column (6 mL volume, 1 g sorbents), Silica gel bonded cartridges (Dikma ProElut Si, 6-mL volume, 1 g sorbents), is activated with 2-5 mL of n-hexane, the eluent is discarded, and then the redissolved lipid solution is poured into the column;
elution of nonpolar lipids: firstly, 1-5 mL of n-hexane/diethyl ether (8:2, V/V) solution are added to the column, and the eluent is collected, then 2-5 mL of n-hexane/diethyl ether (1:1, V/V) solution are added to the column, and the eluent is collected; the eluent is dried with nitrogen to obtain glycerolipids/fatty acids;
elution of phospholipids: 2-5 mL of methanol is added to the column, followed by adding 1-3.5 mL of methanol and 1-3.5 mL of dichloromethane/methanol/water (3:5:2, V/V/V), then the eluent is collected, and the collected eluent is dried with nitrogen to obtain phospholipids.

The method of the present invention can simultaneously extract fatty acids, glycerolipids, phospholipids and gangliosides in food, simultaneously achieve efficient extraction and separation of lipids, thus achieving the effect of less sample consumption, high extraction efficiency and separation of different lipids.

Also disclosed herein, but not being part of the claimed invention, is a method for extracting lipids in food, which can extract fatty acids/glycerides/phospholipids, and gangliosides, respectively, the method comprising the following steps:
1) 100-350 µL of liquid or 0.05-0.15 g powder solid food is sampled to 1# centrifuge tube, followed by adding the mixed internal standard equivalent to the content of each lipid in the food, 100-300 µL of ultrapure water, 1-3 mL of methanol and 500-1500 µL of dichloromethane thereto, and the thus obtained mixture is shaken, followed by sonication, then 100-250 µL of ultrapure water and 500-1500 µL of dichloromethane are added to the mixture again and the thus obtained mixture is shaken; the thus obtained liquid is centrifuged to give an upper phase and lower organic phase, respectively;
2) the upper phase in 1# centrifuge tube is removed to 2# centrifuge tube, followed by adding 1-3 mL of dichloromethane thereto, then the thus obtained mixture is centrifuged to give an upper phase and lower organic phase, respectively;
3) the lower organic phase in 1# centrifuge tube is removed to 3# centrifuge tube, followed by adding 0.5-3 mL of ultrapure water, 1-3.5mL of methanol and 0.3-1.0 mL of dichloromethane thereto, and the thus obtained mixture is shaken, then the obtained liquid is centrifuged to give an upper phase and lower organic phase, respectively;
4) the upper phases from 2# and 3# centrifuge tubes are combined, and dried with nitrogen to obtain the gangliosides;
5) the lower organic phases from 2# and 3# centrifuge tubes are combined, and dried with nitrogen to obtain the phospholipids, fatty acids, glycerolipids.

Also disclosed herein, but not being part of the claimed invention, is a method for extracting lipids in food, which can extract fatty acids/glycerides, phospholipids, and gangliosides, respectively, the method comprising the following steps:
1) 100-350 µL of liquid or 0.05-0.15 g powder solid food is sampled to 1# centrifuge tube, followed by adding the mixed internal standard equivalent to the content of each lipid in the food, 100-350 µL of ultrapure water, 1-3.5 mL of methanol and 500-1500 µL of dichloromethane thereto, and the thus obtained mixture is shaken, followed by sonication, then 100-350 µL of ultrapure water and 500-1500 µL of dichloromethane are added to the mixture again and the thus obtained mixture is shaken; the obtained liquid is centrifuged to give an upper phase and a lower organic phase, respectively;
2) the upper phase in 1# centrifuge tube is removed to 2# centrifuge tube, followed by adding 1-3 mL of dichloromethane thereto, then the thus obtained mixture is centrifuged to give an upper phase and a lower organic phase, respectively;
3) the lower organic phase in 1# centrifuge tube is removed to 3# centrifuge tube, followed by adding 0.5-3 mL of ultrapure water, 1.5-3 mL of methanol and 0.3-1.0 mL of dichloromethane thereto and the thus obtained mixture is shaken, then the obtained liquid is centrifuged to give an upper phase and a lower organic phase, respectively;
4) the upper phases from 2# and 3# centrifuge tubes are combined, and dried with nitrogen to obtain gangliosides;
5) the lower organic phases from 2# and 3# centrifuge tubes are combined, and dried with nitrogen to obtain the phospholipids, fatty acids, and glycerolipids;
6) the phospholipids, fatty acids and glycerolipids obtained in step 5) are dissolved into 0.5-2.5 mL of dichloromethane and methanol (v/v=2:1) to obtain redissolved lipid solution, which is then separated with a silica gel column to obtain the glycerolipids/fatty acids and the phospholipids, respectively.

Also disclosed herein, but not being part of the claimed invention, is a method for detecting lipids in food, which can specifically detect phospholipids/glycerides/fatty acids and gangliosides.

In one implementation, the method for detecting lipids in food comprises the following steps:
1) detection of phospholipids/glycerolipids/fatty acids: lipids or glycerolipids/fatty acids and phospholipids extracted by the above method for extracting lipids in food are taken for dilution to be between the quantitatively detectable upper limit and lower limit for lipids, and detected by LC-Q-TOF-MS (AB Sciex TripleTOF^{®} 6600) under the following conditions: chromatographic column: Kinetex C18 100 Å column (150 mm × 4.6 mm, 2.6 µm; Phenomenex, Torrance, CA, USA) or Kinetex C18 100 Å column (50 mm × 3 mm, 2.6 µm; Phenomenex, Torrance, CA, USA); column temperature: 30-50°C; mobile phase A: 5 nmol/L ammonium acetate solution (methanol\acetonitrile\water, 1\1\1, V\V\V), mobile phase B: 5 nmol/L ammonium acetate solution (isopropanol), flow rate: 0.3-0.8mL/min, injection volume: 1-3 µL.

In one implementation, in case that the detection object is the lipids extracted by the above method for extracting lipids in food, in the method for detecting lipids in food, the elution gradient is performed according to the following table, in order to detect phospholipids/glycerides/fatty acids:

### Detection of lipids simultaneously

| Time | Flow (mL/min) | A% | B.% |
|---|---|---|---|
| | 0.80 | 50.0 | 50.0 |
| 1.00 | 0.80 | 50.0 | 50.0 |
| 10.00 | 0.80 | 2.0 | 98.0 |
| 12.00 | 0.80 | 2.0 | 98.0 |
| 12.10 | 0.80 | 50.0 | 50.0 |
| 15.00 | 0.80 | 50.0 | 50.0 |

In one implementation, in case that the detection object is phospholipids and/or glycerolipids/fatty acids extracted by the above method for extracting lipids in food, in the method for detecting lipids in food, the elution gradient is performed according to the following table:

### Method for detecting glycerolipids/fatty acids/phospholipids

| Time | Flow (mL/min) | A% | B% |
|---|---|---|---|
| 0.00 | 0.80 | 50 | 50 |
| 1.00 | 0.80 | 50 | 50 |
| 10.00 | 0.80 | 2 | 98 |
| 15.00 | 0.80 | 2 | 98 |
| 15.10 | 0.80 | 50 | 50 |
| 20.00 | 0.80 | 50 | 50 |

When a sample has higher contents of phospholipids and glycerolipids, a switching valve can be used after the liquid chromatography column. After a certain retention time, when the phospholipids are separated, triglyceride eluted from the liquid chromatography column is discharged into the waste liquid tank so as to avoid entering into and polluting the mass spectrum by excessive ion concentration.

Detection by mass spectrometry is performed in positive ion mode and negative ion mode, respectively, in the positive and negative ion modes, the parent ion detection parameters are: accumulation time: 0.15 sec, scanning range (tof masses): 100-1300 Da, elution time (duration): 15 min, heating gas (GS1): 50-65 psi, auxiliary heating gas (GS2): 50-65 psi, curtain gas (CUR): 30-45 psi, ion source temperature (TEM): 500-650 °C, ion spray voltage (ISVF): 4500-5500 V (in case of negative ion mode, only changing this parameter to -4500 V), de-clustering voltage (DP): 80 V, collision energy (CE): 10 V. The parameters for secondary fragment detection by mass spectrometry are: accumulation time: 0.05 sec, scanning range (tof masses): 50-1300 Da, heating gas (GS1): 50-65 psi, auxiliary heating gas (GS2): 50-65 psi, curtain gas (CUR): 30-45 psi, ion source temperature (TEM): 500-650°C, ion spray voltage (ISVF): 4500-5500 V (in case of negative ion mode, only changing this parameter to -4500 V), collision energy (CE): 30-50V, collision energy superposition (CES): 10-25 V.

In one implementation, the method for detecting lipids in food comprises the following steps:
2) detection for gangliosides: gangliosides extracted by the above method for extracting lipids in food are taken for dilution to be between the quantitatively detectable upper limit and lower limit of lipids, and detected by LC-Q-TOF-MS (AB Sciex TripleTOF^{®} 6600) under the following conditions: chromatographic column: BEH C18 (100×2.1 mm, 1.7 um; Waters), column temperature: 30-50°C; mobile phase A: 90 mL water+10 mL methanol+0.1mL of 1mol/L ammonium acetate, mobile phase B: 100 mL methanol +0.1 mL of 1mol/L ammonium acetate, flow rate: 0.3 mL/min, injection volume: 10 µL.

In one implementation, in the method for detecting lipids in food, the elution gradient is performed according to the following table, for the detection of gangliosides:

| | Time (min) | Flow rate (mL/min) | Phase A ratio | Phase B ratio |
|---|---|---|---|---|
| 1 | 0 | 0.30 | 10.0 | 90.0 |
| 2 | 0.13 | 0.30 | 10.0 | 90.0 |
| 3 | 3.13 | 0.30 | 5.0 | 95.0 |
| 4 | 4.13 | 0.30 | 5.0 | 95.0 |
| 5 | 6.27 | 0.30 | 0.0 | 100.0 |
| 6 | 12.27 | 0.30 | 0.0 | 100.0 |
| 7 | 12.40 | 0.30 | 10.0 | 90.0 |

Mass spectrometry is performed in negative ion mode, and the parent ion detection parameters are: accumulation time: 0.25 sec, scanning range (tof masses): 100-2000 Da, elution time (duration): 14 min, heating gas (GS1): 35-50 psi, auxiliary heating gas (GS2): 30-45 psi, curtain gas (CUR): 10-25 psi, ion source temperature (TEM): 300-450°C, ion spray voltage (ISVF): -3500-4500 V, de-clustering voltage (DP): -30-50 V, collision energy (CE): -35-50 V. The parameters for secondary fragment detection by mass spectrometry are: accumulation time: 0.05 sec, scanning range (tof masses): 50-2000 Da, heating gas (GS1): 35-50 psi, auxiliary heating gas (GS2): 30-45 psi, curtain gas (CUR): 10-25 psi, ion source temperature (TEM): 300-450 °C, ion spray voltage (ISVF): -3500-4500 V, de-clustering voltage (DP): -35-50 V, collision energy (CE): -35-45 V, collision energy superposition (CES): 15-30 V, ion release delay (IRD): 30, ion release width (IRW): 15.

Also disclosed herein, but not being part of the claimed invention, is a method for qualitative analysis of lipids, wherein the qualitative analysis adopts Peakview 2.2 software, wherein, the method for qualitative analysis of lipids comprises the following steps:
1) qualitative analysis of fatty acids: in negative ion mode, after setting the adduct ion to -H, the ion information is directly compared with the fatty acid database for determination.
2) qualitative analysis of glycerolipids: glyceride is present as the positive ion of +NH₄⁺ in positive ion mode; according to the glyceride database provided by this software, firstly, qualitative analysis is carried out according to the accurate m/z value of the parent ion, and then the composition of fatty acid is determined according to the information of secondary fragments (diglycerides). If the fatty acids qualified by fragments can be combined to give the numbers of carbon atoms and double bonds in the parent ion glyceride, then qualitation of the glyceride can be determined;
3) qualitative analysis of phospholipids: for different types of phospholipids, different qualitative methods are employed, wherein, PA, PG, PI, PS and PE can be firstly carried out on qualitative analysis in negative ion mode based on the parent ion according to imported corresponding data from the database provided by Peakview, with the adduct ion being set to -H (for PC, the adduct ion is set to CH₃COO⁻), and then whether the composition of fatty acid is consistent with the numbers of carbon atoms and double bonds in the corresponding parent ion is determined according to the fragments, if so, then qualitation of the phospholipid can be determined. SM and Cer are analyzed in positive ion mode, when the adduct ion of SM is H⁺,184 secondary characteristic fragments are used for qualitative analysis, and 264 secondary characteristic fragments are used for qualitative analysis of Cer;
4) qualitative analysis of gangliosides: gangliosides are searched in negative ion mode according to the precise molecular weight, isotope and molecular formula according to imported corresponding data from the gangliosides database provided by Peakview, and then whether the searched gangliosides contain a particular ganglioside is determined based on whether sialic acid fragments (the mass-to-charge ratio is 290.1) are present in the secondary fragments.

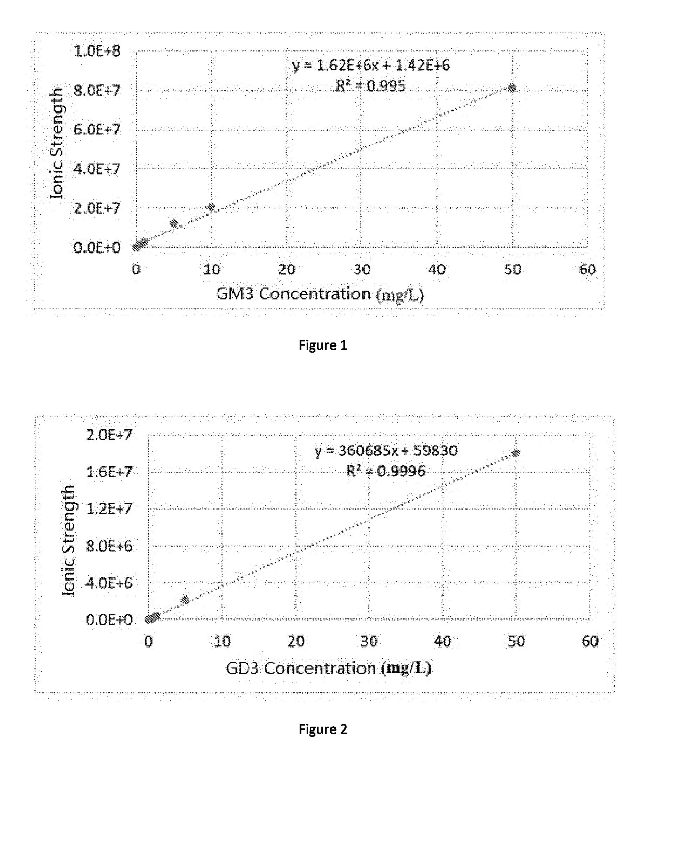

Also disclosed herein, but being part of the claimed invention, is a method for quantitative analysis of lipids, wherein the quantitative analysis adopts MultieQuant 3.0.2 for quantitative analysis of different lipids. Wherein, the method for quantitative analysis of lipids comprises the following steps:
1) quantitative analysis of glycerolipids: because the lipid pretreatment process needs manual operation, resulting unstable random errors, the internal standard method is mainly used to quantify different glycerolipids, wherein, the internal standard contains a deuterated internal standard mixture of 9 types of glycerolipids, and the amount added is determined according to the content of the corresponding glycerolipids in the tested dairy products, based on the principle that the content of the internal standard is similar to that of the corresponding glyceride in the dairy products, the contents of other glycerolipids are calculated by choosing the deuterated internal standard with similar retention time. At the same time, the external standard method is used to quantitatively calculate the contents of different glycerolipids in a sample by establishing a standard curve, with the area ratios of different representative external standard products to internal standard as ordinate and different external standard concentrations as abscissa.
2) quantitative analysis of phospholipids: quantitative analysis of different types of phospholipids in breast milk is performed by both of the internal standard method and external standard method. According to the internal standard method, phosphatidylcholine (PC) 31:2 (17:1;14:1), phosphatidylserine (PS) 31:2 (17:0-14:1), phosphatidylinositol (PI) 31:2 (17:0-14:1), phosphatidylethanolamine (PE) 31:2 (17:0-14:1), phosphatidylglycerol (PG) 31:1 (17:0-14:1), phosphatidic acid (PA), sphingomyelin (SM) d18:1/17:0, and ceramide (d18:2 (4E,8Z) /24:0) are used as internal standards. At the same time, for each kind of polar lipid, one phospholipid is selected therefrom as external standard to quantitatively calculate the contents of different phospholipids in a sample by establishing a standard curve with the area ratios of external standard to corresponding internal standard as ordinate and external standard concentration as abscissa;
3) quantitative analysis of gangliosides: gangliosides are mainly quantified on monosialoganglioside (GM3) and disialoganglioside (GD3) with higher contents, by external standard method. Both GM3 and GD3 standards contain a variety of monomers consisting of different alkyl hydrocarbons and sugars at the same time, so the sum of the peak areas with higher contents is used as the basis for quantitative calculation. Gangliosides in a given sample are quantified, and also the corresponding contents are calculated by the different sums of GM3 and GD3. The standard curves of ganglioside GM3 and GD3 are shown in Fig. 1 and Fig. 2, respectively.

Through the same detection platform LC-Q-TOF-MS (AB Sciex TripleTOF^{®} 6600), non-polar glycerolipids, polar phospholipids and gangliosides are detected by mass spectrometry after elution by suitable chromatographic column, mobile phase and elution gradient, which is the key to simultaneously detect glycerolipids, phospholipids and gangliosides with one injection. The present disclosure establishes two methods, one method can simultaneously detect glycerolipids, fatty acids, phospholipids, and gangliosides, which is used to detect samples containing different types of lipids at the same time, or only detect glycerolipids and/or phospholipids; the other method can only detect gangliosides, which is used to detect the extracted gangliosides samples.

### Advantageous effect

The advantages of the present invention are mainly shown in the following aspects:
1. Glycerolipids, fatty acids, phospholipids and gangliosides can be extracted by one sampling and treatment.
   In the prior art in literatures, gangliosides are extracted separately, and co-extraction of phospholipids, glycerolipids and fatty acids is reported in only a few literatures. According to the present invention, ganglioside extract and extract of phospholipid/fatty acid/glyceride can be obtained respectively according to the need of detection and analysis through one sampling and treatment; based on the concentrations of phospholipids, fatty acids and glycerolipids in a given sample, glycerolipids, fatty acids and phospholipids can be separated to dilute or concentrate to different times for further detection in order to improve the accuracy of qualitative and quantitative analysis of phospholipids, fatty acids and glycerolipids and reduce the pollution to instruments.
2. According to the present invention, dichloromethane is used instead of chloroform to extract lipids, which improves the safety of the experiment.
   Dichloromethane has the least toxic among methane chlorides, and has a toxicity only 0.11% of that of chloroform.
   In the methods reported in literatures, according to the conventional Folch method, 60-80 mL of chloroform is needed for a given sample, and it is necessary to shake and centrifuge in the extraction process, and carry out elution with chloroform in the process of solid phase extraction, which are difficult to be operated in a totally enclosed way. Therefore, in the process of mass sample detection, in addition to the adverse effect on the health of operators and laboratory staff, waste chloroform can cause secondary toxicity in the storage, transportation and treatment thereof. In the present invention, dichloromethane is used instead of chloroform used in the conventional method.
3. A method for simultaneously detecting glycerolipids, phospholipids and gangliosides by LC-Q-TOF-MS is established.
   As reported in literatures, in most cases, different lipids are detected separately, however, the present disclosure establishes a method for simultaneously detecting glyceride, phospholipid, ceramide and sphingolipid by LC- Q-TOF-MS, and establishes a detection method suitable for ganglioside extract individually.
4. This method is suitable for different foods, including liquid and solid forms, especially breast milk, dairy products, and corresponding ingredients.

The reported methods mostly relate to the comparison of phospholipids or glycerolipids or gangliosides in different raw materials, and the pretreatment methods suitable for various raw materials have not been reported yet.

### Brief Description of the Drawings

Fig. 1 is a standard curve of ganglioside GM3, with the concentration as abscissa and the ionic strength as ordinate.
Fig. 2 is a standard curve of ganglioside GD3, with the concentration as abscissa and the ionic strength as ordinate.

### Detailed Description of the Invention

Exemplary embodiments of the present invention are provided in the following examples. The following examples are given by way of example only and are intended to help ordinary technicians to use the present invention. The examples are not intended to limit the scope of the present invention in any way.

### Experimental Example 1: Precision of detection method

During the day and at night, the samples were injected for 6 times with the time interval of 1 h, to determine the precision of the method, and the relative standard deviations (RSD) of different concentrations were as follows:

**Table 2: Precision of detection method**

| | Concentration (mg/L) | Relative standard deviation (RSD) % | |
|---|---|---|---|
| | | Night | Day |
| TAG 52:2 | 0.002 | 30.00 | 12.00 |
| | 0.2 | 5.00 | 10.00 |
| | 2 | 7.00 | 19.00 |
| PC 36:2 | 0.1 | 2.84 | 3.83 |
| | 1 | 3.91 | 2.9 |
| | 10 | 3.42 | 4.75 |
| PE 36:2 | 0.1 | 3.32 | 4.38 |
| | 1 | 5.63 | 5.27 |
| | 5 | 2.12 | 3.77 |
| LPE 18:0 | 0.1 | 4.98 | 5.88 |
| | 1 | 2.15 | 1.95 |
| | 10 | 2.27 | 3.25 |
| Cer 42:1;2 | 0.5 | 12.23 | 5.96 |
| | 100 | 3.18 | 3.15 |
| SM 42:2;2 | 0.01 | 6 | 15.14 |
| | 0.5 | 3.02 | 8.76 |
| | 100 | 4.09 | 8.85 |
| GM3 | 0.5 | 3.16 | 2.05 |
| | 5 | 2.36 | 1.96 |
| | 50 | 6.86 | 2.47 |
| GD3 | 0.5 | 2.89 | 2.16 |
| | 5 | 4.19 | 2.73 |
| | 50 | 6.98 | 7.93 |

### Accuracy

When glycerolipids/phospholipids and gangliosides are extracted separately, the recovery of all the lipids except gangliosides is reasonable (Table 3), which can be directly quantified by external standard method without adding internal standard. In the method for extraction of other two types of lipids, it is necessary to add internal standard for quantitative analysis of contents of various lipids in the sample. The relative content of ganglioside can be calculated.

**Table 3: Recovery**

| | OPO | PC | PE | SM | CER |
|---|---|---|---|---|---|
| Recovery rate (%) | 80-92 | 85-105 | 80-100 | 70-85 | 70-100 |

### Limit of detection and limit of quantitation

**Table 4: Limit of detection and limit of quantitation**

| | OPO | PC | PE | LPE | SM | CER | GM3 | GD3 |
|---|---|---|---|---|---|---|---|---|
| Limit of Detection mg/L | 3×10⁻⁶ | 5× 10⁻⁴ | 1×10⁻³ | 5×10⁻⁴ | 5×10⁻⁴ | 8×10⁻⁴ | 2×10⁻⁴ | 4×10⁻³ |
| Limit of Quantitation mg/L | 1×10⁻⁵ | 1×10⁻³ | 2.5×10⁻³ | 1×10⁻³ | 1.5×10⁻³ | 1×10⁻² | 5×10⁻⁴ | 6×10⁻³ |

### Example 1: Separate extraction of glycerolipids, fatty acids, phospholipids, and gangliosides from breast milk

### 1. Sample pretreatment

1# tube: 200 µL breast milk + 200 µL ultrapure water + 1.8mL methanol+800 µL dichloromethane (chromatographically pure) → manually shaking for 10 sec, sonication for 5 min → adding 200 µL ultrapure water + 800 µL dichloromethane (chromatographically pure) → manually shaking for 10 sec → centrifuging at 6000 r/min for 15 min → removing the lower organic phase to 2# tube →
1) the upper phase in 1# tube + 1.6 mL dichloromethane → centrifuging at 6000 r/min for 15 min → removing the lower organic phase to 3# tube
2) the lower organic phase in 2# tube +1 mL ultrapure water +2.2 mL methanol + 0.6 mL dichloromethane, manually shaking → centrifuging at 3000 g/min for 10 min→ removing the lower organic phase to tube 3# and mixing → drying with nitrogen → dissolving into 1 mL of dichloromethane: methanol (v/v=2:1) for seperation of glycerolipids, fatty acids, and phospholipids by solid phase extraction:
   Activation of silica gel column: the silica gel column (6 mL volume, 1 g sorbents), Silica gel bonded cartridges (Dikma ProElut Si, 6-mL volume, 1 g sorbents) was activated with 3 mL of n-hexane, the eluent was discarded, and then the redissolved lipid solution was poured into the column;
   Elution of nonpolar lipids: firstly, 3 mL of n-hexane/diethyl ether (8:2, V/V) solution was added to the column, and the eluent was collected; then 3 mL of n-hexane/diethyl ether (1:1, V/V) solution were added to the column, and the eluent was collected; the eluent was dried with nitrogen and then redissolved with 2 mL of reconstitution fluid, 100 µL was taken and 900 µL of reconstitution fluid was added thereto. The thus obtained mixture was centrifuged at 6000 r/min for 5 min, the supernatant was collected for machine test of glycerolipids and free fatty acids.
   Elution of phospholipids: 4 mL of methanol was added to the column, followed by adding 2 mL of methanol and 2 mL of dichloromethane/methanol/water (3:5:2, V/V/V), then the eluent was collected, and the collected eluent was dried with nitrogen. 200 µL of reconstitution fluid was added to the thus obtained residue to from mixture which was centrifuged at 6000 r/min for 5 min to collect the supernatant for machine test of phospholipids.
3) The upper phase in 1# tube and the upper phase in 2# tube were combined and dried with nitrogen; The thus obtained residue was redissolving with 200 µL of 80% methanol to form mixture which was centrifuged at 6000 r/min for 5 min to collect the supernatant for machine test.

2. Liquid phase elution: Kinetex C18 100 Å column (150 mm× 4.6 mm, 2.6 µm; Phenene X, Torrance, CA, USA) column was used to detect phospholipids at column temperature of 35 °C, with liquid phase gradient elution.

**Table 5: Elution gradient of Example 1**

| Time | Flow (mL/min) | A% | B% |
|---|---|---|---|
| 0.00 | 0.80 | 50 | 50 |
| 1.00 | 0.80 | 50 | 50 |
| 10.00 | 0.80 | 2 | 98 |
| 15.00 | 0.80 | 2 | 98 |
| 15.10 | 0.80 | 50 | 50 |
| 20.00 | 0.80 | 50 | 50 |

Kinetex C18 100 Å column (50 mm× 3.0 mm, 2.6 µm; Phenene X, Torrance, CA, USA) column was used to detect triglycerides at the following condition: column temperature: 35 °C, flow rate: 0.6 mL/min, liquid phase gradient elution: 0 min, 50%A; 2 min, 50%A; 3 min, 2%A; 8 min, 2%A; 8.1 min, 50%A; 10min, 50%A.

### 3. Mass spectrometric detection

Mass spectrometric detection was performed in positive ion mode and negative ion mode, respectively. In the positive and negative ion modes, the parent ion detection parameters were as follows: accumulation time: 0.15 sec, scanning range (tof masses): 100-1300 Da, elution time (duration): 15 min, heating gas (GS1): 60 psi, auxiliary heating gas (GS2): 60 psi, curtain gas (CUR): 35 psi, ion source temperature (TEM): 600 °C, ion spray voltage (ISVF): 5500 V (in case of negative ion mode, only changing this parameter to -4500 V), de-clustering voltage (DP): 80 V, collision energy (CE): 10 V. The parameters for secondary fragment detection by mass spectrometry were as follows: accumulation time: 0.05 sec, scanning range (tof masses): 50-1300 Da, heating gas (GS1): 60 psi, auxiliary heating gas (GS2): 60 psi, curtain gas (CUR): 35 psi, ion source temperature (TEM): 600 °C, ion spray voltage (ISVF): 5500 V (in case of negative ion mode, only changing this parameter to -4500 V), collision energy (CE): 40V, collision energy superposition (CES): 20 V.

### 4. Analysis of mass spectrometry results

Qualitative analysis of various lipids and the composition of their fatty acid was performed together with relative quantitative analysis of the contents of various lipids.
1) 21 types of free fatty acids were detected, and the fatty acids with higher content were 18:2 (31.39%), 18:1 (31.22%), 14:0 (12.12%), 16:0 (9.83%) and 18:0 (7.84%);
2) 46 types of diglycerides and 97 types of triglycerides were detected, and the diglycerides and the triglycerides with higher content were TAG 52:3 (9.57%, 18:2-18:1-16:0), TAG 52:2 (7.87%, 18:2-18:0-16:0), TAG 54:5 (6.46%,18:2-18:2-18:1), TAG 52:4 (6.25%,18:2-18:2-16:0), TAG 54:4 (6.18%, 18:1-18:1-18:2).
3) Glycerol phospholipids detected included 12 types of phosphatidylcholines, 2 types of lysophosphatidylcholines, 24 types of phosphatidylethanolamines, 8 types of lysophosphatidylethanolamines, 7 types of phosphatidylinositols, 1 type of lysophosphatidylinositol, 4 types of phosphatidic acids, 1 type of phosphatidylglycerol and 7 types of phosphatidylserines. The glycerol phospholipids with higher content were PE 36:2 (35.45%, 18:0-18:2), PS 36:2 (10.63%, 18:0-18:2), PE 36:1 (9.25%, 18:0-18:1), PC 36:2 (8.37%, 18:0-18:2), PE 36:3 (4.20%, 18:1-18:2).
4) For sphingolipids, 19 types of sphingomyelins and 11 types of ceramides were detected, and the sphingolipids with higher content were SM 40:1;2 (31.74%), SM 42:1;2 (30.72%), SM 42:2;2 (13.47%), SM 38:1;2 (8.56%), SM 36:1;2 (5.14%).
5) 25 types of glycolipids were detected, and the glycolipid with higher content were GM3 36:1;2 (20.25%), GM3 40:1;2 (16.79%), GM3 38:1;2 (16.76%), GM3 42:2;2 (16.20%), GM3 34:1;2 (10.17%).

### Example 2: Simultaneous extraction of lipids from breast milk

### 1. Sample pretreatment

80 µL breast milk + 1200 µL ultrapure water + 2.5 mL methanol + 1200 µL dichloromethane (chromatographically pure) → manually shaking for 10 sec, standing for 30 min→ adding 1.5 mL ultrapure water +1 mL dichloromethane (hromatographically pure) → manually shaking for 10 sec → centrifuging at 6000 r/min for 15 min → removing the lower organic phase → the upper phase + 1.6 mL dichloromethane → centrifuging at 6000 r/min for 15 min → removing the lower organic phase and mixing it with the previous one → dred with nitrogen → dissolving with 250 µL of 10mmol/L ammonium acetate solution (v/v, dichloromethane: methanol = 1:1) → centrifuging at 6000 r/min for 5 min.

2. Kinetex C18 100 Å column (150 mm× 4.6 mm, 2.6 µm; Phenene X, Torrance, CA, USA) column was used at column temperature of 40 °C with liquid phase gradient elution.

**Table 6: Liquid elution gradient of Example 2**

| | Time | Flow (mL/min) | A.Conc | B.Conc |
|---|---|---|---|---|
| 1 | | 0.80 | 50.0 | 50.0 |
| 2 | 1.00 | 0.80 | 50.0 | 50.0 |
| 3 | 10.00 | 0.80 | 2.0 | 98.0 |
| 4 | 12.00 | 0.80 | 2.0 | 98.0 |
| 5 | 12.10 | 0.80 | 50.0 | 50.0 |
| 6 | 15.00 | 0.80 | 50.0 | 50.0 |

### 3. Mass spectrometric detection

Mass spectrometric detection was performed in positive ion mode and negative ion mode, respectively, in the positive and negative ion modes, the parent ion detection parameters were as follows: accumulation time: 0.15 sec, scanning range (tof masses): 100-1300 Da, elution time (duration): 15 min, heating gas (GS1): 55 psi, auxiliary heating gas (GS2): 50 psi, curtain gas (CUR): 30 psi, ion source temperature (TEM): 550 °C, ion spray voltage (ISVF): 5000 V, de-clustering voltage (DP): 70 V, collision energy (CE): 10 V. The parameters for secondary fragment detection by mass spectrometry were as follows: accumulation time: 0.05 sec, scanning range (tof masses): 50-1300 Da, heating gas (GS1): 55 psi, auxiliary heating gas (GS2): 50 psi, curtain gas (CUR): 30 psi, ion source temperature (TEM): 550 °C, ion spray voltage (ISVF): 5000 V, collision energy (CE): 35 V, collision energy superposition (CES): 15V.

### 4. Analysis of mass spectrometry results (relative quantitative results were required)

Finally, 7 types of free fatty acids, 76 types of glycerolipids (2 types of monoglycerides, 15 types of diglycerides and 59 types of triglycerides), 42 types of phospholipids (12 types of PCs, 3 types of PAs, 10 types of PEs, 4 types of PGs, 1 type of PS, 8 types of SMs and 4 types of Cers), and 2 types of glycolipids were detected.

### Example 3: Separate extraction of glycerolipids/fatty acids/phospholipids and gangliosides from breast milk and detection therefor in two seperate testing procedures

### 1. Sample pretreatment

1# tube: 100 µL breast milk+150 µL ultrapure water +1.5 mL methanol+800 µL dichloromethane (chromatographically pure) → manually shaking for 10 sec, sonication for 5 min→ adding 200 µL ultrapure water+800 µL dichloromethane (chromatographically pure) → manually shaking for 10 sec → centrifuging at 6000 r/min for 15 min → removing the upper phase to 2# tube:
Step 1: 2# tube +1.6 mL dichloromethane →centrifuging at 6000 r/min for 15 min→ removing the upper phase to 3# tube to be mixed with the upper phase removed from step 2
Step 2: the remaining organic phase in 1# tube +2 mL methanol + 0.8 mL dichloromethane +1 mL ultrapure water → centrifuging at 6000 r/min for 15 min → removing the upper phase and mixing it with the upper phase in 3# tube from step 1, then directly filtering the mixed upper phase and sampling for detecting gangliosides
Step 3: removing the organic phase from 1# tube in step 2 and mixing it with the organic phase from 2# tube → drying with nitrogen → dissolving with a certain amount of 10 mmol/L ammonium acetate solution (V/V, dichloromethane: methanol =1:1) → centrifuging at 6000 r/min for 5 min, and collecting the supernatant for detection of phospholipids, fatty acids and glycerolipids.

2. Kinetex C18 100 Å column (150 mm× 4.6 mm, 2.6 µm; Phenene X, Torrance, CA, USA) column was used at column temperature of 40 °C with liquid phase gradient elution.

**Table 7: Elution gradient of Example 3**

| Time | Flow (mL/min) | A% | B% |
|---|---|---|---|
| 0.00 | 0.80 | 50 | 50 |
| 1.00 | 0.80 | 50 | 50 |
| 10.00 | 0.80 | 2 | 98 |
| 15.00 | 0.80 | 2 | 98 |
| 15.10 | 0.80 | 50 | 50 |
| 20.00 | 0.80 | 50 | 50 |

### 3. Mass spectrometric detection

Mass spectrometric detection was performed in positive ion mode and negative ion mode, respectively, in the positive and negative ion modes, the parent ion detection parameters were as follows: accumulation time: 0.15 sec, scanning range (tof masses): 100-1300 Da, elution time (duration): 15 min, heating gas (GS1): 65 psi, auxiliary heating gas (GS2): 65 psi, curtain gas (CUR): 40 psi, ion source temperature (TEM): 650 °C, ion spray voltage (ISVF): 5500 V, de-clustering voltage (DP): 70 V, collision energy (CE): 15 V. The parameters for secondary fragment detection by mass spectrometry were as follows: accumulation time: 0.05 sec, scanning range (tof masses): 50-1300 Da, heating gas (GS1): 65 psi, auxiliary heating gas (GS2): 65 psi, curtain gas (CUR): 35 psi, ion source temperature (TEM): 600 °C, ion spray voltage (ISVF): 5500 V (in case of negative ion mode, only changing this parameter to -4500 V), collision energy (CE): 40 V, collision energy superposition (CES): 20 V.

### 4. Analysis of mass spectrometry results

Finally, 24 types of free fatty acids, 144 types of glycerolipids (44 types of diglycerides and 100 types of triglycerides), 57 types of phospholipids (10 types of PCs, 27 types of PEs, 3 types of PAs, 2 types of PGs, 11 types of PIs, 4 types of PSes), 22 types of sphingolipids, and 18 types of glycolipids were detected.

### Example 4: Separate extraction of glycerolipids/fatty acids/phospholipids and gangliosides from milk

### 1. Sample pretreatment

1# tube: 100 µL cow milk+150 µL ultrapure water +1.5 mL methanol+800 µL dichloromethane (chromatographically pure) → manually shaking for 10 sec, sonication for 5 min→ adding 200 µL ultrapure water + 800 µL dichloromethane (chromatographically pure) → manually shaking for 10 sec→ centrifuging at 6000 r/min for 15 min→ removing the upper phase to 2# tube:
Step 1: 2# tube + 1.6 mL dichloromethane → centrifuging at 6000 r/min for 15 min→ removing the upper phase to 3# tube to be mixed with the upper phase removed from step 2.
Step 2: the remaining organic phase in 1# tube +2 mL methanol + 0.8 mL dichloromethane +1 mL ultrapure water → centrifuging at 6000 r/min for 15 min → removing the upper phase and mixing it with the upper phase in 3# tube from step 1, then directly filtering the mixed upper phase and sampling for detecting gangliosides
Step 3: removing the organic phase from 1# tube in step 2 and mixing it with the organic phase from 2# tube → drying with nitrogen → dissolving with a certain amount of 10 mmol/L ammonium acetate solution (V/V, dichloromethane: methanol =1:1) → centrifuging at 6000 r/min for 5 min, and collecting the supernatant for detection of phospholipids, fatty acids, and glycerolipids

2. Kinetex C18 100 Å column (150 mm× 4.6 mm, 2.6 µm; Phenene X, Torrance, CA, USA) column was used at column temperature of 35 °C with liquid phase gradient elution.

**Table 8: Elution gradient of Example 4**

| Time | Flow (mL/min) | A% | B% |
|---|---|---|---|
| 0.00 | 0.80 | 50 | 50 |
| 1.00 | 0.80 | 50 | 50 |
| 10.00 | 0.80 | 2 | 98 |
| 15.00 | 0.80 | 2 | 98 |
| 15.10 | 0.80 | 50 | 50 |
| 20.00 | 0.80 | 50 | 50 |

### 3. Mass spectrometric detection

Mass spectrometric detection was performed in positive ion mode and negative ion mode, respectively, in the positive and negative ion modes, the parent ion detection parameters were as follows: accumulation time: 0.15 sec, scanning range (tof masses): 100-1300 Da, elution time (duration): 15 min, heating gas (GS1): 65 psi, auxiliary heating gas (GS2): 65 psi, curtain gas (CUR): 40 psi, ion source temperature (TEM): 650 °C, ion spray voltage (ISVF): 5500 V, de-clustering voltage (DP): 70 V, collision energy (CE): 15 V. The parameters for secondary fragment detection by mass spectrometry were as follows: accumulation time: 0.05 sec, scanning range (tof masses): 50-1300 Da, heating gas (GS1): 65 psi, auxiliary heating gas (GS2): 65 psi, curtain gas (CUR): 35 psi, ion source temperature (TEM): 600 °C, ion spray voltage (ISVF): 5500 V (in case of negative ion mode, only changing this parameter to -4500 V), collision energy (CE): 40 V, collision energy superposition (CES): 20 V.

### 4. Analysis of mass spectrometry results

1) 6 types of free fatty acids were detected, and the free fatty acids with higher content were 18:0 (60.91%), 16:0 (31.84%), 18:1 (4.96%).
2) 8 types of diglycerides and 64 types of triglycerides were detected, and the diglycerides and the triglycerides with higher content were: TAG 36:0 (8.65%, 4:0-14:0-18:0), TAG 38:1(8.00%, 4:0-16:0-18:1), TAG 38:0 (6.60%, 4:0-16:0-18:0), TAG 50:1 (4.99%, 18:1-14:0-18:0), TAG 40:1 (4.95%, 6:0-16:0-18:1).
3) For phospholipids, 6 types of phosphatidylethanols and one type of phosphatidylcholine were detected, and the phospholipids with higher content were PE 36:1 (29.88%, 18:1-18:0), PE 36:2 (23.24%, 18:1-18:1), PE 36:3 (18.75%, 18:1-18:2).
4) Sphingolipids and glycolipids were not detected.

### Example 5: Separate extraction of glycerolipids/fatty acids/phospholipids and gangliosides from milk powder

### 1. Sample pretreatment

Dissolving 4.3 g milk powder in water at 50 °C and shaking for sufficiently dissolving, removing 100 µL to 1# tube + 150 µL ultrapure water +1.5 mL methanol+800 µL dichloromethane (chromatographically pure) → manually shaking for 10 sec, sonication for 5 min→ adding 200 µL ultrapure water + 800 µL dichloromethane (chromatographically pure) → manually shaking for 10 sec→ centrifuging at 6000 r/min for 15 min→ removing the upper phase to 2# tube:
Step 1: 2# tube +1.6 mL dichloromethane → centrifuging at 6000 r/min for 15 min→ removing the upper phase to 3# tube to be mixed with the upper phase removed from step 2.
Step 2: the remaining organic phase in 1# tube +2 mL methanol + 0.8 mL dichloromethane +1 mL ultrapure water → centrifuging at 6000 r/min for 15 min → removing the upper phase and mixing it with the upper phase in 3# tube from step 1, then directly filtering the mixed upper phase and sampling for detecting gangliosides
Step 3: removing the organic phase from 1# tube in step 2 and mixing it with the organic phase from 2# tube → drying with nitrogen → dissolving with a certain amount of 10 mmol/L ammonium acetate solution (V/V, dichloromethane: methanol =1:1) → centrifuging at 6000 r/min for 5 min, and collecting the supernatant for detection of phospholipids, fatty acids, and glycerolipids.

2. Kinetex C18 100 Å column (150 mm× 4.6 mm, 2.6 µm; Phenene X, Torrance, CA, USA) column was used at column temperature of 35 °C with liquid phase gradient elution.

**Table 9: Elution gradient of Example 5**

| Time | Flow (mL/min) | A% | B% |
|---|---|---|---|
| 0.00 | 0.80 | 50 | 50 |
| 1.00 | 0.80 | 50 | 50 |
| 10.00 | 0.80 | 2 | 98 |
| 15.00 | 0.80 | 2 | 98 |
| 15.10 | 0.80 | 50 | 50 |
| 20.00 | 0.80 | 50 | 50 |

### 3. Mass spectrometric detection

Mass spectrometric detection was performed in positive ion mode and negative ion mode, respectively, in the positive and negative ion modes, the parent ion detection parameters were as follows: accumulation time: 0.15 sec, scanning range (tof masses): 100-1300 Da, elution time (duration): 15 min, heating gas (GS1): 65 psi, auxiliary heating gas (GS2): 65 psi, curtain gas (CUR): 40 psi, ion source temperature (TEM): 650 °C, ion spray voltage (ISVF): 5500 V, de-clustering voltage (DP): 70 V, collision energy (CE): 15 V. The parameters for secondary fragment detection by mass spectrometry were as follows: accumulation time: 0.05 sec, scanning range (tof masses): 50-1300 Da, heating gas (GS1): 65 psi, auxiliary heating gas (GS2): 65 psi, curtain gas (CUR): 35 psi, ion source temperature (TEM): 600 °C, ion spray voltage (ISVF): 5500 V (in case of negative ion mode, only changing this parameter to -4500 V), collision energy (CE): 40 V, collision energy superposition (CES): 20 V.

### 4. Analysis of mass spectrometry results

1) 6 types of free fatty acids were detected in milk powder, the free fatty acids with higher content were: 18:0 (61.26%), 16:0 (33.23%).
2) 12 types of diglycerides and 75 types of triglycerides were detected, the glycerolipids with higher content were TAG 52:2 (8.97%, 18:1-16:0-18:1), TAG 54:5 (7.61%, 18:2-18:2-18:1), TAG 50:1 (7.41%, 16:0-16:0-18:1), TAG 54:6 (7.02%, 18:3-18:1-18:2), TAG 54:3 (6.36%, 18:2-18:2-18:0).
3) 4 types of phosphatidylcholines, 6 types of phosphatidylethanolamines and 4 types of phosphatidylinositols were detected, the phospholipids with higher content were: PE 36:2 (36.49%, 18:1-18:1), PE 36:3 (12.64%, 18:1-18:2) and PE 34:1 (10.44%, 16:0-18:1).
4) Sphingolipids and glycolipids were not detected.

### Comparative Example 1

Compared with the method used in Example 1, this comparative example used the conventional Folch method for extraction, which was specifically as follows:
100 µL breast milk +2.5 mL chloroform/methanol solution (v/v=2:1) + 250 µL NaCl (0.05M) were, shaken in a shaker at 200 rpm for 20 min, and then centrifuged at 5000 rpm for 20 min. The bottom chloroform was collected, and 1.8 mL of chloroform was added to the upper layer, the thus obtained mixture was shaken in a shaker at 200 rpm for 20 min and centrifuged at 5000 rpm for 20 min. The bottom chloroform was collected and combined with the previously collected one. The thus obtained mixture was dried with nitrogen, then the solide residue was subjected to dissolution and solid phase extraction according to the method of Example 1 to give phospholipids, fatty acids, and glycerolipids for detection. And the upper layer was dried with nitrogen, and the redissolution and centrifugation were performed according to the method of Example 1 to give gangliosides for detection. The results obtained from the same breast milk sample were compared in the following table. It can be seen from the table that the types of free fatty acids, glycerolipids, glycerophospholipids, sphingolipids and gangliosides extracted by Folch method were less than those extracted by the method according to the present invention..

| | Method according to Example 1 | Method according to Comparative Example 1 |
|---|---|---|
| Free fatty acids detected | 21 | 9 |
| Diglyceride | 18 | 3 |
| Triglyceride | 70 | 25 |
| Phosphatidylcholine | 9 | 5 |
| Phosphatidylethanolamine | 35 | 12 |
| Phosphatidylinositol | 9 | 2 |
| Phosphatidic acid | 9 | 4 |
| phosphatidylglycerol | 2 | 0 |
| Phosphatidylserine | 7 | 3 |
| Sphingomyelin | 19 | 14 |
| Ceramide | 7 | 0 |
| Ganglioside | 14 | 4 |

### Industrial applicability

The present invention provides a method for extracting lipids in food and a method for detecting lipids in food. The method for extracting lipids in food comprising: mixing food samples with mixed internal standard, followed by mixing with lipid extraction solvent and ultrapure water and then centrifugating, which process is repeated several times, then combining the upper phases comprising gangliosides which are collected after several centrifugations, and combining the lower organic phases comprising phospholipids, fatty acids, and glycerolipids which are collected after several centrifugations. By using small sample size and safer lipid extraction reagents, the method according to the present invention can obtain extract of different lipids by one-time treatment, which then can be detected qualitatively and quantitatively, simultaneously or separately. Therefore, the method according to the present invention can reduce sample consumption, improve safety, save consumables in sample pretreatment and detection, reduce cost and improving efficiency, and has application prospect.

## Claims

1. A method for extracting lipids in food, comprising steps of:
(1) mixing a food sample with mixed internal standard, wherein the mixed internal standard is one or more of deuterated glycerolipids, different types of deuterated phospholipids, and different types of deuterated sphingolipids;
the mixed internal standard is used in an amount such that the mass concentration of each lipid after adding the mixed internal standard is 60-140% of the mass concentration of the same type of lipid in the original sample;
(2) mixing the mixture of the food sample and the mixed internal standard with a mixture of dichloromethane and methanol as a lipid extraction solvent and adding ultrapure water for performing a first centrifugation to obtain an upper phase and a lower organic phase, wherein the volume ratio of dichloromethane, methanol and ultrapure water is 1-3.5:1-3:0.2-0.55; dichloromethane is used in an amount of 0.5-2 mL/100 µL liquid food sample or 1-3 mL/0.05 g powdered solid food sample;
(3) adding dichloromethane to the upper phase obtained by the first centrifugation for performing a second centrifugation to obtain an upper phase and a lower organic phase; dichloromethane is used in an amount of 0.2-1.8 mL/100 µL liquid food sample or 1-3 mL/0.05 g powdered solid food sample; adding ultrapure water, methanol, and dichloromethane to the lower organic phase obtained by the first centrifugation for performing a third centrifugation to obtain an upper phase and a lower organic phase; the volume ratio of ultrapure water, methanol and dichloromethane is 0.3-1:1-3.5:0.5-3; dichloromethane is used in an amount of 0.3-1 mL/100 µL liquid food sample or 0.3-1 mL/0.05 g powdered solid food sample;
(4) combining the upper phase obtained by the second centrifugation with the upper phase obtained by the third centrifugation, and drying the combined upper phases with nitrogen to obtain gangliosides;
combining the lower organic phase obtained by the second centrifugation with the lower organic phase obtained by the third centrifugation, and drying the combined lower organic phases with nitrogen to obtain phospholipids, glycerolipids, and fatty acids; and
(5) dissolving the obtained phospholipids, fatty acids, and glycerolipids in the solution of dichloromethane and methanol with a volume ratio of 0.6-1.5:0.6-1.2 to obtain a redissolved lipid solution, and then separating the redissolved lipid solution with a silica gel column to obtain glycerolipids/fatty acids and phospholipids, respectively;
the solution of dichloromethane and methanol is used in an amount of 0.2-2.5 mL/100 µL liquid food sample or 0.5-2.5 mL/0.05 g powderedsolid food sample;
wherein, the lipids are gangliosides, phospholipids, glycerolipids, and fatty acids.

2. The method according to claim 1, wherein in step (2), the volume ratio of dichloromethane, methanol and ultrapure water is 1-3:1-3:0.3-0.55; and
wherein dichloromethane is used in an amount of 0.6-1.2 mL/100 µL liquid food sample or 1.5-2.5 mL/0.05 g powdered solid food sample.

3. The method according to claim 2, wherein in step (3), dichloromethane is used in an amount of 0.6-1.2 mL/100 µL liquid food sample or 1.5-2.5 mL/0.05 g powdered solid food sample;
the volume ratio of ultrapure water, methanol and dichloromethane is 0.3-0.6:1-2:0.5-1.

4. The method according to claim 3, further comprising steps of purifying the gangliosides by dissolving the obtained gangliosides in a 60-90% methanol solution, followed by centrifugation, then taking the upper phase for drying under nitrogen to obtain gangliosides; preferably, the methanol solution is used in an amount of 100-350 µL/100 µL liquid food sample or 100-350 µL/0.05 g powdered solid food sample.

5. The method according to claim 3, further comprising steps of purifying phospholipids, glycerolipids, and fatty acids by dissolving the obtained phospholipids, glycerolipids, and fatty acids in a solution of ammonium acetate in dichloromethane and methanol, followed by centrifugation, then by taking the lower organic phase and drying it with nitrogen to obtain the phospholipids, glycerolipids and fatty acids;
preferably, the ratio of dichloromethane and methanol in the solution of ammonium acetate in dichloromethane and methanol is 0.6-1.5:0.6-1.2; more preferably 1:1; the concentration of ammonium acetate is 5-15 mmol/L, preferably 8-12 mmol/L; the solution of ammonium acetate in dichloromethane and methanol is used in an amount of 0.2-2.5 mL/100 µL liquid food sample or 0.5-2.5 mL/0.05 g powdered solid food sample.

## Patentansprüche

1. Verfahren zur Extraktion von Lipiden in Lebensmitteln, das folgende Schritte umfasst:
(1) Mischen einer Lebensmittelprobe mit einem gemischten internen Standard, wobei der gemischte interne Standard eines oder mehrere der folgenden Elemente ist:
deuterierte Glycerolipide; verschiedene Arten von deuterierten Phospholipiden; und
verschiedene Arten von deuterierten Sphingolipiden;
der gemischte interne Standard in einer solchen Menge verwendet wird, dass die Massenkonzentration jedes Lipids nach Zugabe des gemischten internen Standards 60-140% der Massenkonzentration der gleichen Lipidart in der ursprünglichen Probe beträgt;
(2) Mischen des Gemischs aus der Lebensmittelprobe und dem gemischten internen Standard mit einem Gemisch aus Dichlormethan und Methanol als Lipidextraktionslösungsmittel und Hinzufügen von ultrareinem Wasser zur Durchführung einer ersten Zentrifugation, um eine obere Phase und eine untere organische Phase zu erhalten, wobei das Volumenverhältnis von Dichlormethan, Methanol und ultrareinem Wasser 1-3,5:1-3:0,2-0,55 beträgt;
wobei Dichlormethan in einer Menge von 0,5-2 mL/100 µL flüssiger Lebensmittelprobe oder 1-3 mL/0,05 g pulverisierter fester Lebensmittelprobe verwendet wird;
(3) Zugeben von Dichlormethan zu der durch die erste Zentrifugation erhaltenen oberen Phase zur Durchführung einer zweiten Zentrifugation, um eine obere Phase und eine untere organische Phase zu erhalten; wobei Dichlormethan in einer Menge von 0,2-1,8 mL/100 µL flüssiger Lebensmittelprobe oder 1-3 mL/0,05 g pulverisierter fester Lebensmittelprobe verwendet wird;
Zugeben von Reinstwasser, Methanol und Dichlormethan zu der durch die erste Zentrifugation erhaltenen unteren organischen Phase zur Durchführung einer dritten Zentrifugation, um eine obere Phase und eine untere organische Phase zu erhalten;
wobei das Volumenverhältnis von Reinstwasser, Methanol und Dichlormethan 0,3-1:1-3,5:0,5-3 beträgt; wobei Dichlormethan in einer Menge von 0,3-1 mL/ 100 µL flüssiger Lebensmittelprobe oder 0,3-1 mL/0,05 g pulverisierter fester Lebensmittelprobe verwendet wird;
(4) Zusammenführen der durch die zweite Zentrifugation erhaltenen oberen Phase mit der durch die dritte Zentrifugation erhaltenen oberen Phase; und Trocknen der zusammengeführten oberen Phasen mit Stickstoff, um Ganglioside zu erhalten;
Zusammenführen der durch die zweite Zentrifugation erhaltenen unteren organischen Phase mit der durch die dritte Zentrifugation erhaltenen unteren organischen Phase und Trocknen der zusammengeführten unteren organischen Phasen mit Stickstoff, um Phospholipide, Glycerolipide und Fettsäuren zu erhalten; und
(5) Auflösen der erhaltenen Phospholipide, Fettsäuren und Glycerolipide in der Lösung von Dichlormethan und Methanol mit einem Volumenverhältnis von 0,6-1,5:0,6-1,2, um eine wiedergelöste Lipidlösung zu erhalten, und dann Trennen der wiedergelösten Lipidlösung mit einer Kieselgelsäule, um Glycerolipide/Fettsäuren bzw. Phospholipide zu erhalten;
wobei die Lösung aus Dichlormethan und Methanol in einer Menge von 0,2-2,5 mL/100 µL flüssiger Lebensmittelprobe oder 0,5-2,5 mL/0,05 g pulverförmiger fester Lebensmittelprobe verwendet wird;
wobei die Lipide Ganglioside, Phospholipide, Glycerolipide und Fettsäuren sind.

2. Verfahren nach Anspruch 1, wobei in Schritt (2) das Volumenverhältnis von Dichlormethan, Methanol und Reinstwasser 1-3:1-3:0,3-0,55 beträgt; und
wobei Dichlormethan in einer Menge von 0,6-1,2 mL/100 µL flüssiger Lebensmittelprobe oder 1,5-2,5 mL/0,05 g pulverisierter fester Lebensmittelprobe verwendet wird.

3. Verfahren nach Anspruch 2, wobei in Schritt (3) Dichlormethan in einer Menge von 0,6-1,2 mL/100 µL flüssiger Lebensmittelprobe oder 1,5-2,5 mL/0,05 pulverisierter fester Lebensmittelprobe verwendet wird;
wobei das Volumenverhältnis von Reinstwasser, Methanol und Dichlormethan 0,3-0,6:1-2:0,5-1 beträgt.

4. Verfahren nach Anspruch 3, das ferner die Schritte der Reinigung der Ganglioside durch Auflösen der erhaltenen Ganglioside in einer 60-90%igen Methanollösung, gefolgt von Zentrifugation, dann Entnehmen der oberen Phase zum Trocknen unter Stickstoff, um Ganglioside zu erhalten, umfasst; wobei vorzugsweise die Methanollösung in einer Menge von 100-350 µL/100 µL flüssiger Lebensmittelprobe oder 100-350 µL/0,05 g pulverisierter fester Lebensmittelprobe verwendet wird.

5. Verfahren nach Anspruch 3, aufweisend ferner die Schritte des Reinigens von Phospholipiden, Glycerolipiden und Fettsäuren durch Auflösen der erhaltenen Phospholipide, Glycerolipide und Fettsäuren in einer Lösung von Ammoniumacetat in Dichlormethan und Methanol, gefolgt von Zentrifugation, dann durch Entnehmen der unteren organischen Phase und deren Trocknung mit Stickstoff, um die Phospholipide, Glycerolipide und Fettsäuren zu erhalten:
wobei vorzugsweise das Verhältnis von Dichlormethan und Methanol in der Lösung von Ammoniumacetat in Dichlormethan und Methanol 0,6-1,5:0,6-1.2 beträgt; noch bevorzugter 1:1; wobei die Konzentration von Ammoniumacetat 5-15 mmol/L, vorzugsweise 8-12 mmol/L beträgt; wobei die Lösung von Ammoniumacetat in Dichlormethan und Methanol in einer Menge von 0,2-2,5 mL/100 µL flüssiger Lebensmittelprobe oder 0,5-2,5 mL/0,05 g pulverisierter fester Lebensmittelprobe verwendet wird.

## Revendications

1. Un procédé pour extraire des lipides dans de la nourriture, comprenant les étapes suivantes :
(1) le fait de mélanger un échantillon d'aliment avec un agent de référence interne mélangé, l'agent de référence interne mélangé étant un ou plusieurs parmi des glycérolipides deutérés, différents types de phospholipides deutérés et différents types de sphingolipides deutérés ; l'agent de référence interne mélangé est utilisé en une quantité telle que la concentration massique de chaque lipide après addition de l'agent de référence interne mélangé va de 60 à 140% de la concentration massique du même type de lipide dans l'échantillon d'origine ;
(2) le fait de mélanger le mélange de l'échantillon d'aliment et de l'agent de référence interne mélangé avec un mélange de dichlorométhane et de méthanol en tant que solvant d'extraction de lipides, et le fait d'ajouter de l'eau ultrapure pour effectuer une première centrifugation afin d'obtenir une phase supérieure et une phase organique inférieure, le rapport volumique du dichlorométhane, du méthanol et de l'eau ultrapure étant de 1-3,5:1-3:0,2-0,55 ; le dichlorométhane est utilisé en une quantité 0,5 à 2ml/100µl d'échantillon de nourriture liquide ou de 1 à 3ml/0,05g d'échantillon de nourriture solide en poudre ;
(3) le fait d'ajouter du dichlorométhane à la phase supérieure obtenue par la première centrifugation afin de réaliser une deuxième centrifugation pour obtenir une phase supérieure et une phase organique inférieure ;
le dichlorométhane est utilisé en une quantité allant de 0,2 à 1,8ml/100µl d'échantillon de nourriture liquide ou de 1 à 3ml/0,05g d'échantillon de nourriture solide en poudre ;
le fait d'ajouter de l'eau ultrapure, du méthanol et du dichlorométhane à la phase organique inférieure obtenue par la première centrifugation pour effectuer une troisième centrifugation afin d'obtenir une phase supérieure et une phase organique inférieure ;
le rapport volumique de l'eau ultrapure, du méthanol et du dichlorométhane est de 0,3-1:1-3,5:0,5-3 ; le dichlorométhane est utilisé en une quantité 0,3 à 1ml/100µl d'échantillon de nourriture liquide ou de 0,3 à 1ml/0,05g d'échantillon de nourriture solide en poudre ;
(4) le fait de combiner la phase supérieure obtenue par la deuxième centrifugation avec la phase supérieure obtenue par la troisième centrifugation, et de sécher les phases supérieures combinées avec de l'azote pour obtenir des gangliosides ;
le fait de combiner la phase organique inférieure obtenue par la deuxième centrifugation avec la phase organique inférieure obtenue par la troisième centrifugation, et de sécher les phases organiques inférieures combinées avec de l'azote pour obtenir des phospholipides, des glycérolipides et des acides gras ; et
(5) le fait de dissoudre les phospholipides, les acides gras et les glycérolipides obtenus dans la solution de dichlorométhane et de méthanol avec un rapport volumique de 0,6-1,5:0,6-1,2 de manière à obtenir une solution de lipides redissoute, puis le fait de séparer la solution de lipides redissoute au moyen d'une colonne de gel de silice de manière à obtenir respectivement des glycérolipides/acides gras et des phospholipides ;
la solution de dichlorométhane et de méthanol est utilisée en une quantité 0,2 à 2,5ml/100µl d'échantillon de nourriture liquide ou de 0,5 à 2,5ml/0,05g d'échantillon de nourriture solide en poudre ;
les lipides étant des gangliosides, des phospholipides, des glycérolipides et des acides gras.

2. Le procédé selon la revendication 1, dans lequel, à l'étape (2), le rapport volumique du dichlorométhane, du méthanol et de l'eau ultrapure est de 1-3:1-3:0,3-0,55 ; et
dans lequel le dichlorométhane est utilisé en une quantité 0,6 à 1,2ml/100µl d'échantillon de nourriture liquide ou de 1,5 à 2,5ml/0,05g d'échantillon de nourriture solide en poudre.

3. Le procédé selon la revendication 2, dans lequel, à l'étape (3), le dichlorométhane est utilisé en une quantité allant de 0,6 à 1,2ml/100µl d'échantillon de nourriture liquide ou de 1,5 à 2,5ml/0,05g d'échantillon de nourriture solide en poudre ;
le rapport volumique de l'eau ultrapure, du méthanol et du dichlorométhane est de 0,3-0,6:1-2:0,5-1.

4. Le procédé selon la revendication 3, comprenant en outre les étapes consistant à purifier les gangliosides en dissolvant les gangliosides obtenus dans une solution de méthanol à 60 à 90%, puis à centrifuger, puis à prélever la phase supérieure pour la faire sécher sous azote afin d'obtenir des gangliosides ; de préférence, la solution de méthanol est utilisée en une quantité allant de 100 à 350µL/100µL d'échantillon de nourriture liquide ou de 100 à 350µL/0,05g d'échantillon de nourriture solide en poudre.

5. Le procédé selon la revendication 3, comprenant en outre des étapes de purification des phospholipides, des glycérolipides et des acides gras par dissolution des phospholipides, des glycérolipides et des acides gras obtenus dans une solution d'acétate d'ammonium dans le dichlorométhane et le méthanol, suivie d'une centrifugation, puis par prélèvement de la phase organique inférieure et séchage à l'azote pour obtenir les phospholipides, les glycérolipides et les acides gras ;
de préférence, le rapport du dichlorométhane et du méthanol dans la solution d'acétate d'ammonium dans le dichlorométhane et le méthanol est de 0,6-1,5:0,6-1,2 ; de manière encore préférée, de 1:1 ; la concentration d'acétate d'ammonium est de 5 à 15mmol/l, de préférence de 8 à 12mmol/l ; la solution d'acétate d'ammonium dans le dichlorométhane et le méthanol est utilisée en une quantité allant de 0,2 à 2,5ml/100µl d'échantillon de nourriture liquide ou de 0,5 à 2,5ml/0,05g d'échantillon de nourriture solide en poudre.
